# EUROPEAN PATENT SPECIFICATION

(11) **EP 4 577 294 B1**
(45) Date of publication and mention of the grant of the patent: **24.06.2026**
(21) Application number: 22777765.3
(22) Date of filing: 25.08.2022
(51) Int. Cl.: A61N 1/08, A61N 1/36, A61N 1/05

(54) **COCHLEAR IMPLANTS HAVING MRI-COMPATIBLE MAGNET ASSEMBLIES WITH DAMPING LIQUID AND ASSOCIATED METHODS OF ASSEMBLING**
COCHLEA-IMPLANTATE MIT MRI-KOMPATIBLEN MAGNETANORDNUNGEN MIT DÄMPFUNGSFLÜSSIGKEIT UND ZUGEHÖRIGE MONTAGEVERFAHREN
IMPLANTS COCHLÉAIRES AYANT DES ASSEMBLAGES D'AIMANTS COMPATIBLES MRI AVEC UN LIQUIDE D'AMORTISSEMENT ET MÉTHODES D'ASSEMBLAGE ASSOCIÉES

(43) Date of publication of application: 02.07.2025
(73) Proprietor: Advanced Bionics, LLC, Valencia CA 91355 (US)
(72) Inventor: SMITH, James George Elcoate, Santa Clarita, CA 91350 (US); LEE, Sung Jin, Valencia, CA 91354 (US)
(74) Representative: Schwan Schorer & Partner mbB
(86) International application number: PCT/US2022/041589
(87) International publication number: WO 2024/043896

(56) References cited:
- WO-A1-2019/083540
- US-A1- 2019 046 797
- US-B2- 10 532 209
- US-B2- 9 919 154

## Description

### BACKGROUND

### 1. Field

The present disclosure relates generally to the implantable portion of implantable cochlear stimulation (or "ICS") systems.

### 2. Description of the Related Art

ICS systems are used to help the profoundly deaf perceive a sensation of sound by directly exciting the intact auditory nerve with controlled impulses of electrical current. Ambient sound pressure waves are picked up by an externally worn microphone and converted to electrical signals. The electrical signals, in turn, are processed by a sound processor, converted to a pulse sequence having varying pulse widths, rates and/or amplitudes, and transmitted to an implanted receiver circuit of the ICS system. The implanted receiver circuit is connected to an implantable electrode array that has been inserted into the cochlea of the inner ear, and electrical stimulation current is applied to varying electrode combinations to create a perception of sound. The electrode array may, alternatively, be directly inserted into the cochlear nerve without residing in the cochlea. A representative ICS system is disclosed in U.S. Patent No. 5,824,022, which is entitled "Cochlear Stimulation System Employing Behind-The-Ear Sound processor With Remote Control". Examples of commercially available ICS sound processors include, but are not limited to, the Harmony^{™} BTE sound processor, the Naida^{™} CI Q Series sound processor and the Neptune^{™} body worn sound processor, which are available from Advanced Bionics.

As alluded to above, some ICS systems include an implantable cochlear stimulator (or "cochlear implant"), a sound processor unit (e.g., a body worn processor or behind-the-ear processor), and a microphone that is part of, or is in communication with, the sourd processor unit. The cochlear implant communicates with the sound processor unit and, some ICS systems include a headpiece that is in communication with both the sound processor unit and the cochlear implant. The headpiece communicates with the cochlear implant by way of a transmitter (e.g., an antenna) on the headpiece and a receiver (e.g., an antenna) on the implant. Optimum communication is achieved when the transmitter and the receiver are aligned with one another. To that end, the headpiece and the cochlear implant may include respective positioning magnets that are attracted to one another, and that maintain the position of the headpiece transmitter over the implant receiver. The implant magnet may, for example, be located within a pocket in the cochlear implant housing. The skin and subcutaneous tissue that separates the headpiece magnet and implant magnet is sometimes referred to as the "skin flap," which is frequently 3 mm to 11 mm thick.

The present inventors have determined that conventional cochlear implants are susceptible to improvement. For example, the magnets in some conventional cochlear implants are disk-shaped and have north and south magnetic dipoles that are aligned in the axial direction of the disk. Such magnets are not compatible with magnetic resonance imaging ("MRI") systems, and may have to be surgically removed from the cochlear the implant prior to the MRI procedure and then surgically replaced thereafter. Other cochlear implants include with a diametrically magnetized disk-shaped magnet that is rotatable relative to the remainder of the implant about its central axis, and that has a N-S orientation which is perpendicular to the central axis. The present inventors have determined that diametrically magnetized disk-shaped magnets are less than optimal because a dominant magnetic field, such as the MRI magnetic field, that is misaligned by at least 30° or more from the N-S direction of the magnet may demagnetize the magnet or generate an amount of torque on the magnet that is sufficient to dislodge or reverse the magnet and/or dislocate the associated cochlear implant and/or cause excessive discomfort to the patient.

More recently, cochlear implants with MRI-compatible magnet assemblies have been introduced. The MRI-compatible magnet assemblies have a case defining a central axis, a frame within the case that is rotatable relative to the case about the central axis, and a plurality of elongate diametrically magnetized magnets, i.e., diametrically magnetized magnets with respective lengths that are greater than the respective diameters, that are located in the frame and rotatable about their respective longitudinal axis relative to the frame. This combination allows the magnets to align with three-dimensional (3D) MRI magnetic fields, regardless of field direction, which results in very low amounts of torque on the magnets. The case is hermetically sealed by welding (e.g., laser welding) the case cover to the case base around the perimeter of the case after the frame and magnets are positioned therein. Examples of MRI-compatible magnet assemblies may be found in U.S. Pat. Nos. 9,919,154, 10,463,849, and 10,532,209. Another proposed magnet assembly, which includes a single elongate magnet, is described in PCT Pat. Pub. No. 2020/092185 A1.

Although such MRI-compatible magnet assemblies have proven to be a significant advance in the art, the present inventors have determined that they are susceptible to improvement. For example, a relatively small number of recipients of cochlear implants with MRI-compatible magnet assemblies have reported that they hear sound, which may be characterized as a "rattle," when the associated headpiece is attached or removed. For example, some single-sided recipients wear a hearing aid on the contra-lateral side and the hearing aid may amplify sounds emanating from the MRI-compatible magnet assembly. One proposed solution involves hermetically sealing a liquid (e.g., a biocompatible oil) inside the magnet case that acts as a physical dampening medium between the moving internal components of the MRI-compatible magnet assembly. During assembly, the liquid is added to the case base along with the frame and magnets, the case cover is positioned on top of the case base, and the case cover is welded to the case base around the perimeter of the case to hermetically seal the case. The present inventors have determined that hermetically sealing a liquid inside the magnet case in this manner is susceptible to improvement. In particular, adding a volume of liquid sufficient to perform the damping function results in the surface of the liquid being adjacent to the case base and cover intersection where the weld is formed. The high temperature associated with welding (approx. 1700°C) boils the liquid adjacent to the area being welded and adversely effects the weld.
US 2019/046797 A1 relates to a method of assembling a magnet assembly of a cochlear implant, wherein a plurality of elongate diametrically magnetized magnets is positioned within a first case member including a first end wall and a first side wall portion, and a second case member, including a second end wall and a second side wall portion, is secured to the first case member to form a case defining a central axis and in which the elongate diametrically magnetized magnets are located, by welding with a laser welder the first side wall portion to the second side wall portion.

### SUMMARY

The invention relates to a method of assembling a magnet assembly as defined in claim 1, which includes the steps of positioning a plurality of elongate diametrically magnetized magnets within a first case member including a first end wall and a first side wall portion, securing a second case member, including a second end wall and a second side wall portion, to the first case member to form a case, defining a central axis and in which the elongate diametrically magnetized magnets are located, by welding the first side wall portion to the second side wall portion, wherein one of the first and second end walls includes a port, introducing damping liquid into the case by way of the port, sealing the port after the damping liquid into the case has been introduced into the case by way of the port.

A cochlear implant in accordance with the present invention may include a cochlear lead, an antenna, a stimulation processor, and a magnet assembly produced by such methods.

The above described and many other features of the present inventions will become apparent as the inventions become better understood by reference to the following detailed description when considered in conjunction with the accompanying drawings.

### BRIEF DESCRIPTION OF THE DRAWINGS

Detailed descriptions of the exemplary embodiments will be made with reference to the accompanying drawings.
FIG. 1 is a perspective view of an implant magnet assembly in accordance with one embodiment of the present invention.
FIG. 2 is a perspective view of a portion of the implant magnet assembly illustrated in FIG. 1.
FIG. 3 is an exploded perspective view of the implant magnet assembly illustrated in FIG. 1.
FIG. 4 is a top view of a portion of the implant magnet assembly illustrated in FIG. 1.
FIG. 5 is an exploded partial section view of the implant magnet assembly illustrated in FIG. 1 prior to the introduction of damping liquid.
FIG. 6 is an exploded partial section view of the implant magnet assembly illustrated in FIG. 1 prior to the plug being welded to the case.
FIG. 7 is a partial section view taken along line 7-7 in FIG. 1.
FIG. 8 is a flow chart showing a method in accordance with one embodiment of the present invention.
FIG. 9 is an end view of a portion of the implant magnet assembly illustrated in FIG. 1.
FIG. 10 is a perspective view of a portion of the implant magnet assembly illustrated in FIG. 1.
FIG. 11 is a partial section view of a system including a headpiece and an implant with the magnet assembly illustrated in FIG. 1.
FIG. 12 is a partial section view similar to FIG. 11 with the implant in an MRI magnetic field.
FIG. 13 is a perspective view of an implant magnet assembly not in accordance with the present invention.
FIG. 14 is a perspective view of a portion of the implant magnet assembly illustrated in FIG. 13.
FIG. 15 is an exploded perspective view of the implant magnet assembly illustrated in FIG. 13.
FIG. 16 is a top view of a portion of the implant magnet assembly illustrated in FIG. 13.
FIG. 17 is a perspective view of a portion of the implant magnet assembly illustrated in FIG. 13.
FIG. 18 is a perspective view of an exemplary damping liquid dispenser.
FIG. 19 is an exploded perspective view of the exemplary damping liquid dispenser illustrated in FIG. 18.
FIG. 20 is a partial section view of the exemplary damping liquid dispenser illustrated in FIG. 18 in a first orientation.
FIG. 21 is a partial section view of the exemplary damping liquid dispenser illustrated in FIG. 18 in a second orientation that is rotationally offset 90° from the first orientation.
FIG. 22 is a partial section view taken along line 22-22 in FIG. 13 with the exemplary damping liquid dispenser illustrated in FIG. 18 in the first orientation.
FIG. 23 is a partial section view of the exemplary damping liquid dispenser illustrated in FIG. 18 in the first orientation and without the plug.
FIG. 24 is a partial section view of the implant magnet assembly illustrated in FIG. 13 with the exemplary damping liquid dispenser illustrated in FIG. 18 in a third orientation that is rotationally offset 180° from the first orientation.
FIG. 25 is a partial section view of the exemplary damping liquid dispenser illustrated in FIG. 18 in the third orientation and without the plug.
FIG. 26 is a flow chart showing a method not in accordance with the present invention.
FIG. 27 is a top view of a cochlear implant in accordance with one embodiment of the present invention.
FIG. 28 is a block diagram of a cochlear implant system in accordance with one embodiment of the present invention.

### DETAILED DESCRIPTION OF THE EXEMPLARY EMBODIMENTS

The following is a detailed description of the best presently known modes of carrying out the inventions. This description is not to be taken in a limiting sense, but is made merely for the purpose of illustrating the general principles of the inventions.

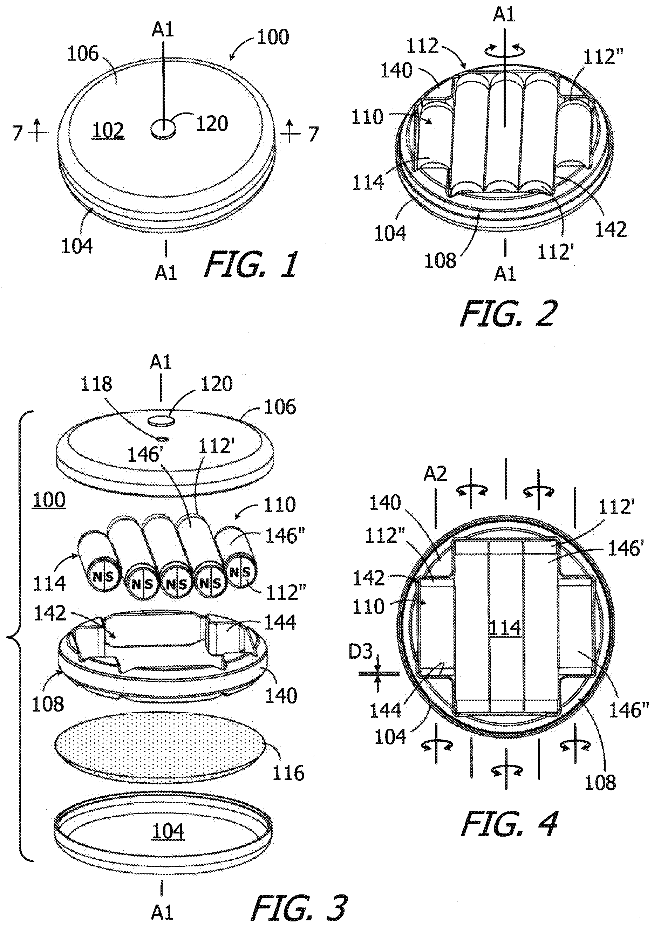

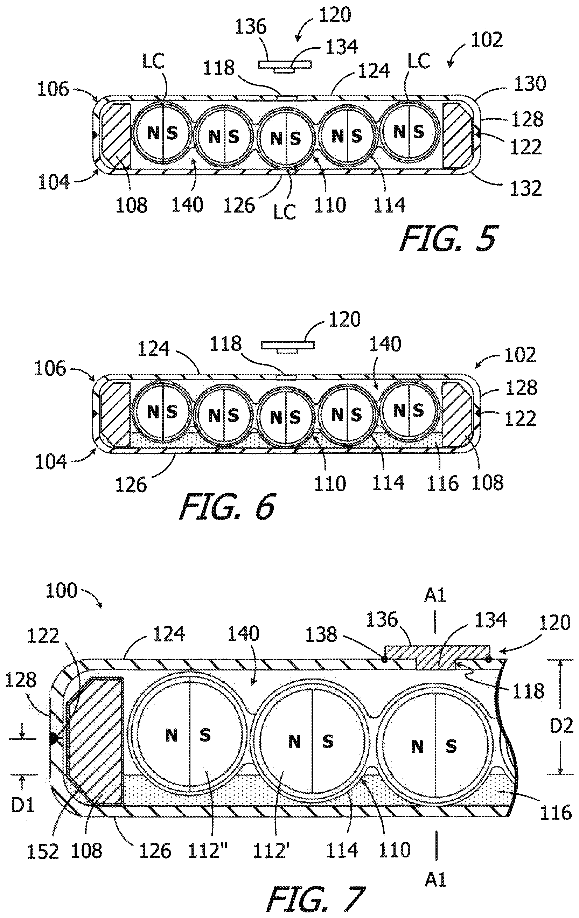

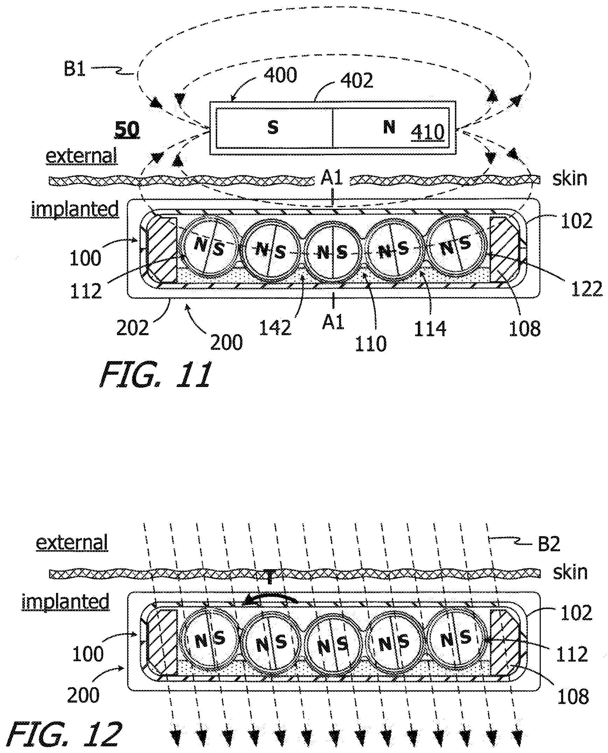

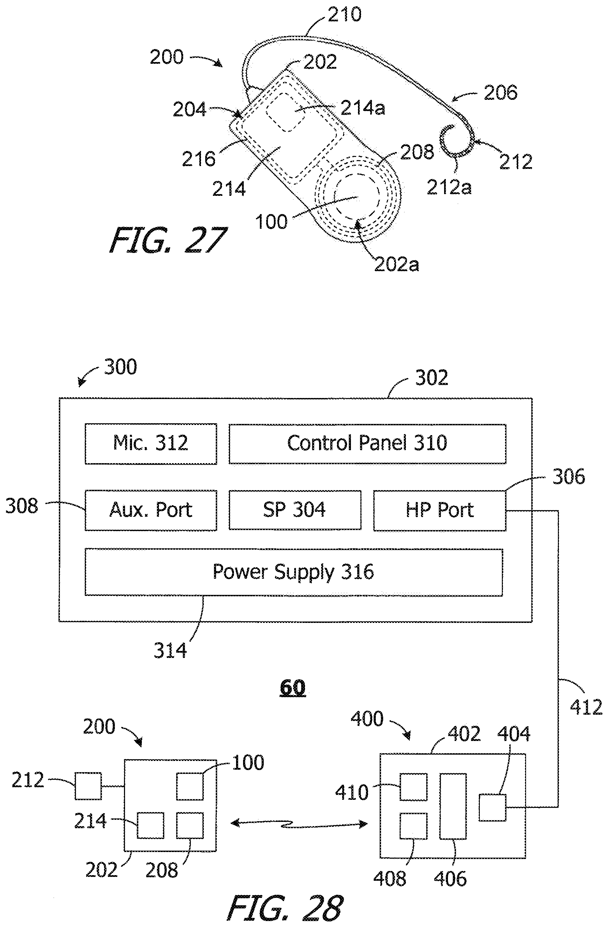

As illustrated for example in FIGS. 1-7, an exemplary magnet assembly 100 includes a case 102, with base 104 and a cover 106, a frame 108, a magnet subassembly 110 within the frame that includes a plurality of elongate diametrically magnetized magnets 112' and 112" (collectively "magnets 112") that define a N-S direction and are located within a holder 114, and damping liquid 116. The damping liquid 116 may be introduced into the case 102 by way of a port 118, and the port 118 may be thereafter sealed with a plug 120 that is welded to the case 102, as is discussed in greater detail below with reference to FIGS. 5-7. The magnet assembly 100 may, in some instances, be employed a system 50 (FIG. 11) that includes a cochlear implant 200 (described below with reference to FIG. 27) with the magnet assembly 100 and an external device such as a headpiece 400 (described below with reference to FIGS. 11 and 28).

The exemplary case 102 is disk-shaped and defines a central axis A1, which is also the central axis of the frame 108. The frame 108 is rotatable relative to the case 102 about the central axis A1 over 360°. The magnet subassembly 110 rotates with the frame 108 about the central axis A1, and does not rotate relative to the frame about the central axis A1. Each magnet 112 is also rotatable within the holder 114 relative to the holder, the case 102 and the frame 108 about its own longitudinal axis A2 (also referred to as "axis A2") over 360°. The N-S direction of each magnet 112 is perpendicular to the longitudinal axis A2 about which the magnet 112 rotates. The holder 114 holds all of the magnets 112 and fixes their positions relative to one another.

In the illustrated implementation, the longitudinal axes A2 are parallel to one another and are perpendicular to the central axis A1. In other implementations, the magnets within a magnet assembly may be oriented such that the longitudinal axes thereof are at least substantially perpendicular to the central axis A1. As used herein, an axis that is "at least substantially perpendicular to the central axis" includes axes that are perpendicular to the central axis as well as axes that are slightly non-perpendicular to the central axis (i.e., axes that are offset from perpendicular by up to 5 degrees).

Given the ability of each magnet 112 to rotate about its longitudinal axis A2, the magnets 112 align with one another in the N-S direction in the absence of an external magnetic field that is strong enough to rotate the magnets out of alignment (e.g., an MRI magnetic field or a headpiece magnetic field). The at rest N-S orientation of the magnets 112 will be perpendicular to the central axis A1 in the illustrated embodiment, as is illustrated in FIGS. 3 and 5-7.

The case 102 is not limited to any particular configuration, size or shape. Referring more specifically to FIGS. 5-7, the exemplary case 102 in the illustrated implementation is, as noted above, a two-part structure that includes the base 104 and the cover 106 which are secured to one another in such a manner that a hermetic seal is formed between the cover and the base. Suitable techniques for securing the cover 106 to the base 104 include, for example, seam welding with a laser welder that creates a weld 122. The completed case 102 has end walls 124 and 126, a side wall 128, and first and second curved walls 130 and 132 that respectively connect the first and second end walls to the side wall. The end walls 124 and 126 are planar, while the side wall 128 is annular in shape and defines the outer perimeter of the case 102. The port 118 may extend through either one of the end walls 124 and 126 and, in the illustrated implementation, extends through end wall 124. The port 118 is located on the case central axis A1, which is at the center of the end wall 124. The diameter of the port 118 may range from, for example, about 0.2 mm to about 1.0 mm. As used herein in the context of dimensions, the word "about" means +/- 10%.

The plug 120 is not limited to any particular configuration, size or shape. In the illustrated implementation, the plug 120 includes a stopper 134 and a flange 136 that extends outwardly from the stopper. Although the stopper 134 and flange 136 are circular in cross-sections perpendicular to the case central axis A1, other shapes may be employed. The outer perimeter of the flange 136, i.e., the diameter in the illustrated embodiment, is also significantly smaller than that of the case 102. For example, the diameter of the flange 136 may range from about 0.3 mm to about 1.5 mm. Alternatively, in those instances where the port 118 is relatively small (e.g., less than 0.3 mm), the port may be sealed by heating the area with a laser spot welder to cause the metal in that portion of the case to liquify and then seal the port when it cools.

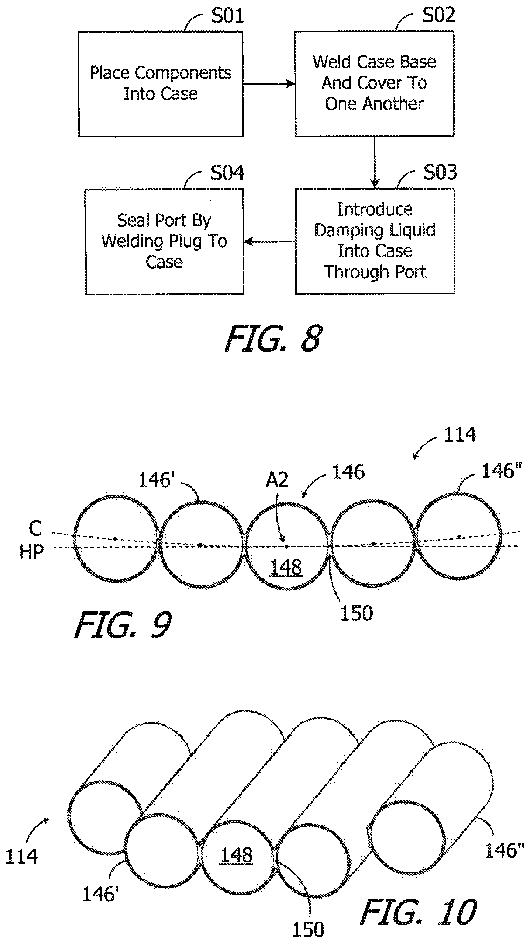

During manufacture of the exemplary magnet assembly 100, the frame 108 and magnet subassembly 110 may be placed into the case base 104, and the case cover 106 may then be positioned on the case base. The case base and cover 104 and 106 may then be secured to one another by a welding process (e.g., laser welding), which results in the weld 122 that hermetically seals the case base and cover to one another to complete the case 102, as shown in FIG. 5. The damping liquid 116 is thereafter introduced into the case 102 through the port 118 and allowed to settle under gravity at the bottom of the case, as shown in FIG. 6. The exemplary plug 120 may then be used to hermetically seal the port 118. In particular, the stopper 134 may be inserted into the port 118 until flange 136 rests on the case end wall 124. The plug 120 may be secured to the case 102 by a welding process (e.g., laser welding) which results in a weld 138 that extends around the perimeter of the flange 136, thereby completing the magnet assembly 100, as shown in FIG. 7. In summary, and turning to FIG. 8, in one exemplary method, the internal components of a magnet assembly may be placed into a first portion of a case (Step S01), a second portion of the case may then be welded to the first portion to complete the case (Step S02), damping liquid may then be introduced into the case through a port in a case end wall (Step S03) and, after the damping liquid has settled under gravity at the bottom of the case, the port may then be sealed with a plug that is welded to the case end wall (Step S04). Subsequent movement of the internal components of the magnet assembly 100, i.e., rotation of the frame 108 relative to the case 102 and/or rotation of the magnets 112 relative the case 102, the frame 108 and the holder 114, will cause the damping liquid to encapsulate the internal components and thereby act as a physical dampening medium between the components.

There are a number of advantages associated with the above-described method of assembling a magnet assembly. By way of example, and referring to FIG. 7, there is a relatively small distance D1 between the location of the weld 122 and the surface of the damping liquid 116. Formation of the weld 122 after the damping liquid 116 has been introduced into the case 102 in accordance with conventional methods will, as noted above, cause the damping liquid to boil in the area adjacent to the area being welded which, in turn, adversely effects the weld. The present method, on the other hand, involves the formation of the weld 122 prior to the introduction of the damping liquid 116, thereby obviating the issues associated with the boiling of damping fluid during the formation of the weld 122. Turning to weld that is formed after the damping fluid has been introduced into the case 102 by way of the port 118, i.e., the weld 138 that secures the plug 120 to the case end wall 124, there is a relatively large distance D2 between the location of the weld 138 and the surface of the damping liquid 116. For example, in some instances, the distance D1 may be about 0.5 mm while the distance D2 may be about 2.3 mm. As a result, the formation of the weld that hermetically seals the case 102 with the damping liquid 116 therein, i.e., weld 138, is far less likely to cause the damping liquid 116 to boil. The likelihood of damping liquid boiling is further reduced by the relatively small size of the weld 138, which extends around the perimeter of the plug flange 136, as compared to the weld 112, which extends around the perimeter of the case 102.

With respect to materials, the case 102, the case 102a (discussed below) may be formed from biocompatible paramagnetic metals, such as titanium or titanium alloys, and/or biocompatible non-magnetic plastics such as polyether ether ketone (PEEK), low-density polyethylene (LDPE), high-density polyethylene (HDPE), ultra-high-molecular-weight polyethylene (UHMWPE), polytetrafluoroethylene (PTFE) and polyamide. In particular, exemplary metals include commercially pure titanium (e.g., Grade 2) and the titanium alloy Ti-6Al-4V (Grade 5), while exemplary metal thicknesses for the case 102 may range from 0.20 mm to 0.25 mm. The plug 120 may be formed from the same material as the case 102.

With respect to size and shape, the exemplary case 102 (and 102a) may have an overall size and shape similar to that of conventional cochlear implant magnets so that the magnet assembly 100 can be substituted for a conventional magnet in an otherwise conventional cochlear implant. The case 102 (and 102a) may also have an overall size and shape that is larger than that of conventional cochlear implant magnets in other embodiments. In some implementations, and depending on the number of magnets within the case 102 (and 102a), the diameter that may range from 9 mm to 17.40 mm and the thickness may range from 1.5 mm to 3.10 mm. The diameter of the case 102 is 15.2 mm, and the thickness is 3.10 mm, in the illustrated embodiment.

Referring to FIGS. 2-4, the exemplary frame 108 includes a disk 140 and a magnet receptacle 142 that extends completely through the disk and is defined by inner walls 144. The magnet receptacle 142 is configured to hold the magnet subassembly 110, including all of the magnets 112 and the holder 114, and has a relatively long portion and two relatively short portions. Suitable materials for the frame 108 (as well as the frame 108a discussed below), which may be formed by machining, metal injection molding or injection molding, include paramagnetic metals, polymers and plastics such as those discussed above in the context of the case 102. Referring more specifically to FIG. 4, there may be a relatively tight fit between the between the magnet subassembly 110 and the magnet receptacle 142. For example, the distance D3 between the longitudinal ends of the magnets 112 and the inner walls 144 may be about 0.08 to 0.15 in some implementations.

Although the present inventions are not limited to any particular number, there are five elongate diametrically magnetized magnets 112 in the exemplary magnet assembly 100 illustrated in FIGS. 1-7. Three magnets 112' are relatively long, as compared to the other magnets, and two magnets 112" are relatively short, as compared to the other magnets, in order to efficiently utilize the available volume within the case 102, as is best shown in FIG. 4. The exemplary magnets 112, which are otherwise identical, are circular in a cross-section that is perpendicular to the longitudinal axis A2 and, in some instances, may have rounded corners. Suitable materials for the magnets 112 include, but are not limited to, neodymium-boron-iron and samarium-cobalt.

Turning to FIGS. 9 and 10, the exemplary holder 114 includes a plurality of tubes 146' and 146" (collectively "tubes 146"), with respective lumens 148, for the plurality of magnets 112. The tubes 146 define the same longitudinal axes A2 as the magnets 112. The number of tubes 146 corresponds to the number of magnets 112 and, in the illustrated embodiment, there are three otherwise identical tubes 146' that are relatively long and two otherwise identical tubes 146" that are relatively short. The exemplary holder 114 is an integral structure wherein the tubes 146 are each attached to an adjacent tube (or tubes). As used herein, an "integral structure" is a structure where adjacent components (i.e., tubes) are attached to one another, remain attached to one another under normal use conditions, and cannot be separated from one another without destruction of the holder. Joints 150 maintain the integrity of the connection between the tubes, and prevent ovaling of the tubes. In some instances, the tubes 146 may share a common wall portion, thereby reducing the overall width of the holder 114.

The magnets 112 are located within the lumens 148 of the exemplary holder 114 in the manner illustrated in FIGS. 2-4 and each magnet 112 rotates about its longitudinal axis A2 relative to the associated tube 146. To facilitate such rotation, the holder 114 may be formed from low friction material including, but not limited to, polymers, such as silicone, PEEK and other plastics, PTFE, and PEEK-PTFE blends, and paramagnet metals. In the illustrated implementation, the holder 114 is a multi-lumen PEEK extrusion where all of the tubes are initially cut to the length of the relatively long tubes 146', and the relatively short tubes 146" are thereafter cut to their length. The diameter of the lumens 148 may be slightly larger (e.g., about 0.05 mm to about 0.2 mm larger) than the outer diameter of the magnets 112 to facilitate placement of the magnets into the holder 114, rotation of the magnets 112 relative to the tubes 146, and the ingress of damping liquid 116 into the space between the tubes and the magnets as the damping liquid encapsulates the internal components of the magnet assembly 100.

Referring more specifically to FIG. 9, it should also be noted that exemplary holder 114 has a pre-set arcuate shape. The longitudinal axis A2 of the center tube 112 lies in a horizontal plane HP and the longitudinal axes A2 of the remaining tubes 112 are not located in the horizontal plane HP when the holder 114 is in a relaxed (i.e., unstressed) state. The axes A2 define a curve C and, as a result, fewer than all of the tubes 112 are in contact with the inner surface of the case 102 when the holder is in a relaxed state. Referring to FIG. 5, there are only three lines of contact LC between the holder 114 due to the curvature and the inner surface of the case 102 as opposed to the minimum of five lines of contact which would be the case if the holder was not arcuate. The reduction in contact results in a corresponding result in friction between the holder 114 and the inner surface of the case 102, as compared to an otherwise identical magnet assembly 100 without an arcuate holder.

Friction may be further reduced by coating the inner surfaces of the case 102 and/or the surfaces of the frame 108 with a lubricious layer. The lubricious layer may be in the form of a specific finish of the surface that reduces friction, as compared to an unfinished surface, or may be a coating of a lubricious material such as diamond-like carbon (DLC), titanium nitride (TiN), PTFE, polyethylene glycol (PEG), Parylene, fluorinated ethylene propylene (FEP) and electroless nickel sold under the tradenames Nedox^{®} and Nedox PF^{™}. The DLC coating, for example, may be only 0.5 to 5 microns thick. In those instances where the base 104 and a cover 106 are formed by stamping, the finishing process may occur prior to stamping. In the illustrated implementation, the surfaces of the frame 108 may be coated with a lubricious layer 152 (e.g., DLC), while the inner surfaces of the case 102 do not include a lubricious layer, as shown in FIG. 7. The lubricious layer 152 reduces friction between the case 102 and frame 108, while the low friction holder 114 reduces friction between adjacent magnets 112 as well as between the case 102 and the magnets 112.

With respect to the damping liquid 116, damping liquids may include, but are not limited to, liquids that are biocompatible and are at least somewhat chemically inert because the liquid will encapsulate the magnets 112. By way of example, but not limitation, the damping liquid may be mineral oil or white cosmetic oil. The damping liquid may have a viscosity that is greater than or equal to about 86 centipoise (cps) and is less than or equal to about 150,000 cps. The volume of damping liquid 116 employed will depend on the particulars of the case, frame and magnets. In the illustrated embodiment, the volume of damping liquid 116 may range from about 20 mm³ to about 70 mm³ in some implementations, may be less than 82 mm³ in some implementations, and is about 30 mm³ in the illustrated implementation.

As shown in FIGS. 5-7, absent an external magnetic field that is strong enough to rotate the magnets 112 out of alignment (e.g., an MRI magnetic field or a headpiece magnetic field), the magnets of the exemplary magnet assembly 100 will remain substantially aligned with one another in the N-S direction and the N-S orientation of each magnet will be perpendicular or close to perpendicular to the central axis A1 of the case 102. The magnets are also arranged an arcuate group defined by the holder 114 in the illustrated embodiment.

Referring to FIG. 11, the exemplary magnet assembly 100 may part of a cochlear implant 200 with a housing 202 (described below with reference to FIG. 27) that is employed in conjunction with an external device such as a headpiece 400 (described below with reference to FIG. 28) in a system 50. The exemplary headpiece 400 includes, among other things, a housing 402 and a magnetized disk-shaped positioning magnet 410. Here, the strength of the dominant headpiece magnetic field B1 causes the magnets 112 to rotate slightly about axis A2 (FIG. 4) from the at rest orientations illustrated in FIGS. 5-7 to the orientations illustrated in FIG. 11. The magnet holder 114 will, as a result of its preset shape, remain unstressed. The frame 108 will also rotate about axis A1 as necessary to align the magnetic fields of the magnets 112 with the N-S direction of the magnetic field B1.

Turning to FIG. 12, when exposed to a dominant MRI magnetic field B2, the torque T on the magnets 112 will rotate the magnets about their axis A2 (FIG. 4), thereby aligning the magnetic fields of the magnets 112 with the MRI magnetic field B2. The frame 108 will also rotate about axis A1 as necessary to align the magnetic fields of the magnets 112 with the MRI magnetic field B2. When the magnet assembly 100 is removed from the MRI magnetic field B2, the magnetic attraction between the magnets 112 will cause the magnets to rotate about axis A2 back to the orientation illustrated in FIGS. 5-7, where they are substantially aligned with one another in the N-S direction and the N-S orientation of the magnets is close to perpendicular to the central axis A1 of the case 102.

It should be noted the exemplary embodiments described above with reference to FIGS. 1-12 may be modified in a variety of ways. By way of example, but not limitation, the number of magnets 112 may be decreased to four or three or two or one, or may be increased to six or more. The holder 114 may be omitted and, in some instances, the magnets 112 may be located within tubes that are formed from low friction material, as is discussed below. A two-piece frame, such that discussed below, may employed in place of the one-piece frame 108.

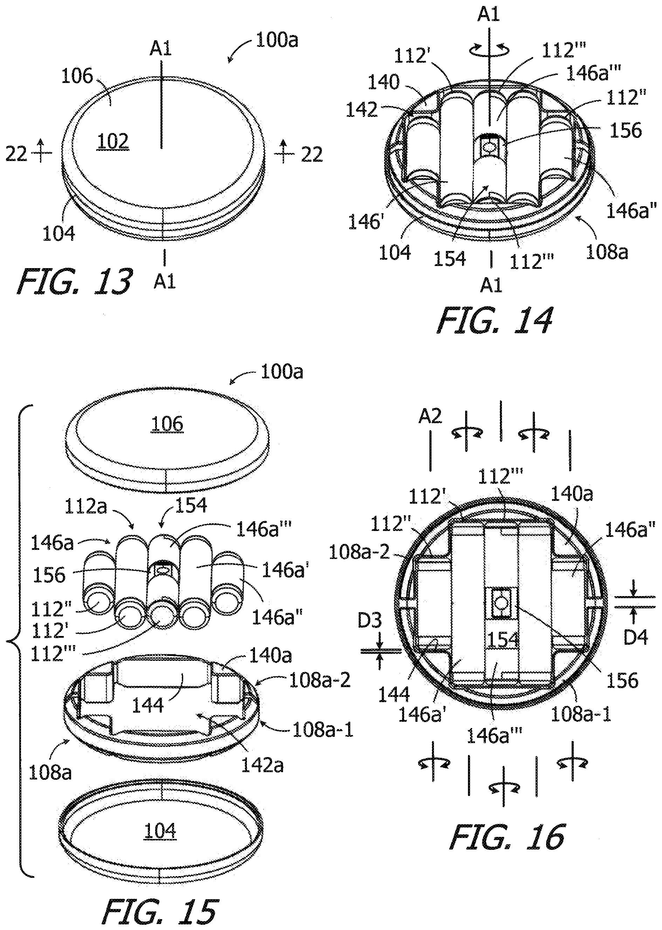

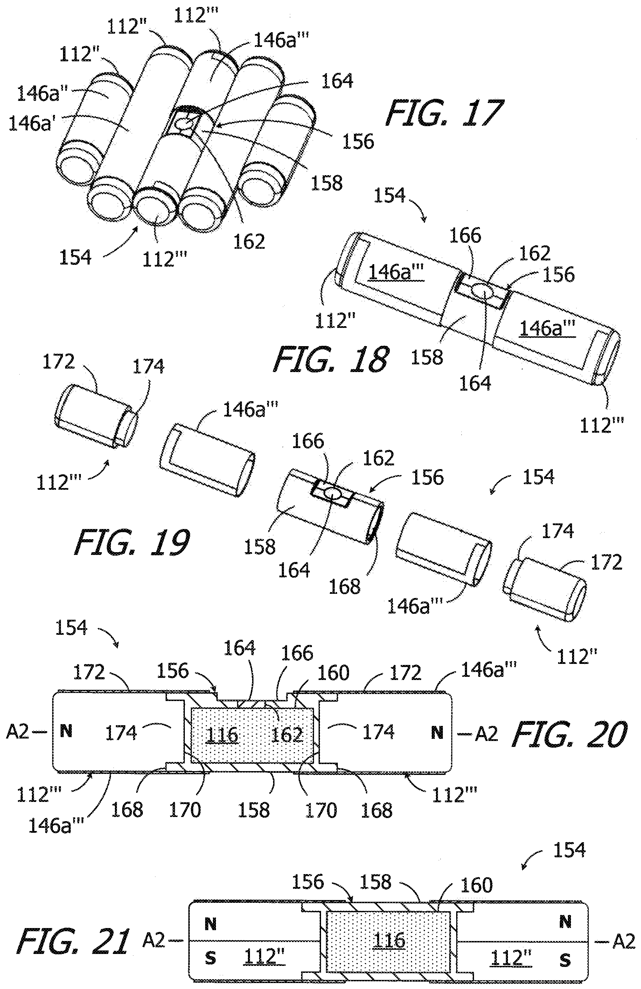

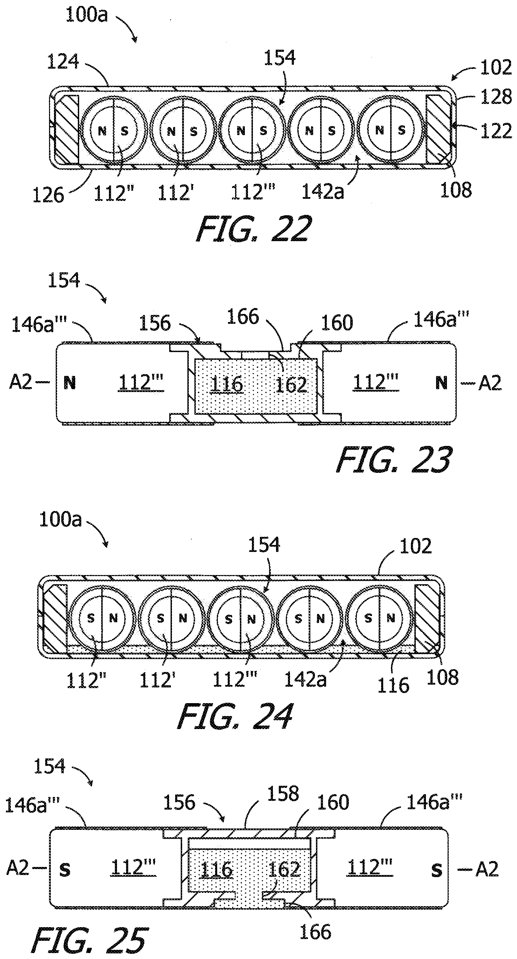

The exemplary magnet assembly 100a illustrated in FIGS. 13-16 and 22 is substantially similar to the magnet assembly 100 and similar elements are represented by similar reference numerals. To that end, the magnet assembly 100a includes a case 102, with a base 104 and a cover 106, a frame 108a, and elongate diametrically magnetized magnets 112' and 112". The cover 106 is secured to the base 104 with a weld 122 (FIG. 22) that extends around the perimeter of the case 102, and the completed case 102 has end walls 124 and 126, a side wall 128. The case 102 is also disk-shaped and defines a central axis A1, which is also the central axis of the frame 108a. The frame 108a is rotatable relative to the case 102 about the central axis A1 over 360°. Here, however, the frame 108a includes two separate frame members 108a1 and 108a2, the holder 114 is omitted, and the magnet assembly includes damping liquid dispenser 154, which is discussed in greater detail below with reference to FIGS. 17-25.

With respect to the magnets, in addition to two of the above-described relatively long diametrically magnetized magnets 112' and two of the above-described relatively short diametrically magnetized magnets 112", the exemplary magnet assembly 100a includes two dispenser magnets 112‴ that are part of the damping liquid dispenser 154. The dispenser magnets 112‴ are also each circular in a cross-section that is perpendicular to the longitudinal axis A2, may have rounded corners, and may be formed from the same material as magnets 112' and 112". The elongate diametrically magnetized magnets 112', 112" and 112'" are referred to collectively "magnets 112a". The magnets 112a rotate with the frame 108a about the central axis A1. Each magnet 112a is rotatable relative to the case 102 and the frame 108a about its own longitudinal axis A2 over 360°. The N-S direction of each magnet 112a is perpendicular to the longitudinal axis A2 about which the magnets 112a rotate in the illustrated embodiment.

The dispenser magnets 112‴ are mounted on opposite ends of a reservoir 156 (discussed below) and the length of the damping liquid dispenser 154 is the same as that of the relatively long magnets 112'. The magnets 112', 112" and 112'" may be located within tubes 146a', 146a" and 146a‴ (collectively "tubes 146a") that are formed from low friction material. Suitable materials for the tubes 146a include polymers, such as silicone, PEEK and other plastics, PTFE, and PEEK-PTFE blends, and paramagnet metals. The magnets 112a are free to rotate relative to the tubes 146a in the illustrated embodiment. The inner diameter of the tubes 146a may be slightly larger (e.g., about 0.05 mm to about 0.2 mm larger) than the outer diameter of the magnets 112a to facilitate placement of the magnets into the tubes, rotation of the magnets relative to the tubes, and ingress of damping liquid 116 into the space between the tubes 146a and the magnets as the damping liquid encapsulates the internal components of the magnet assembly 100a.

Turning to the frame 108a, each of the frame members 108a1 and 108a2 includes a partial disk 140a with inner walls 144 and has an overall C-shape with a curved end and two free ends. When the separate partial disks 140a are positioned adjacent to one another in the manner illustrated in FIGS. 14-16, the partial disks will together define a magnet receptacle 142a that extends completely through the frame 108a and is defined by the inner walls 144. The case 102, the frame 108a and the magnets 112a are respectively sized such that, when the magnets and the damping liquid dispenser 154 are located within the magnet receptacle 142a, the free ends of the frame members 108a1 and 108a2 will face one another and will be separated from one another by a distance D4, while the longitudinal ends of the magnets 112 will be separated from the inner walls 144 by distance D3. Distance D3 may be about 0.08 to 0.15 in some implementations and distance D4 may be about 0.2 to 0.6 in some implementations as noted above. The exemplary magnets 112' and 112" and damping liquid dispenser 154 may be combined with the exemplary frame members 108a1 and 108a2 by placing the magnets, dispenser and frame members on a surface, together with the frame members on opposite sides of the magnets and dispenser, and the frame member inner walls 144 facing the longitudinal ends of the magnets and dispenser. The frame members 108a1 and 108a2 may then be pushed toward one another until the frame members abut the magnets 112' and 112" and damping liquid dispenser 154. The magnets 112' and 112", frame 108a and damping liquid dispenser 154 may then be transferred to the case base 104, and the case cover 106 may thereafter be secured to the case base, by welding or other suitable technique, to complete the magnet assembly 100a.

Turning to FIGS. 17-21, and as noted above, the exemplary damping liquid dispenser 154 includes a reservoir 156 on which the dispenser magnets 112'" are mounted. The exemplary reservoir 156 includes a generally cylindrical reservoir body 158, with an internal storage volume 160, and a port 162 that is temporarily sealed with a plug 164. Damping liquid 116 is initially located within the storage volume 160. The port 162 may be located within a recess 166 that provides clearance to facilitate passage of the damping liquid 116 when the plug 164 is no longer sealing the port 162, as is discussed below. The magnets 112‴ may be connected to the reservoir 156 through the use of any suitable instrumentality. In the illustrated implementation, the reservoir 156 includes a pair of annular flanges 168 (FIG. 20) that project outwardly in the direction of axis A2 and define recesses 170, while the magnets 112‴ each include a cylindrical main portion 172 and a disc-shaped projection 174. The lengths and diameters of the recesses 170 are the same as those of disc-shaped projections 174, which facilitates the close fit illustrated in FIG. 20, and adhesive (not shown) may be used to secure the projections to the reservoir body 158 and flanges 168. The magnets 112‴ also have the same N-S orientation relative to the reservoir 156, i.e. they are N-S aligned with one another and face in the same N-S direction, which allows the magnets to together function as a single elongate diametrically magnetized magnet in the manner described above with reference to magnets 112' and 112".

The exemplary reservoir 156 may, for example, be formed from two molded reservoir halves (not shown) that are ultrasonically welded together. Suitable materials for the reservoir 156 include, but are not limited to, PEEK and other engineering polymers. The internal storage volume 160 may be equal to the intended volume of damping liquid 116 to be dispensed by the dispenser 154 into the interior of the case 102. As such, the size of the internal storage volume 160 will depend on the particulars of the case 102, frame 108a and magnets 112a. In the illustrated embodiment, the size of the internal storage volume 160 (and volume of damping liquid 116) may range from about 7 mm³ to about 20 mm³ in some implementations, may be less than about 25 mm³ in some implementations, and is about 8.6 mm³ in the illustrated implementation.

The exemplary plug 164 is configured to seal the port 162 and prevent the damping fluid 116 from exiting the internal storage volume 160 until the plug is heated to at least a predetermined temperature which causes the plug to shrink or melt. The orientation and/or movement of the magnet assembly 100a during and/or after heating will cause the shrunk or melted plug 164 to fall out of, and thereby open, the port 162. For example, the port may be unsealed by heating the plug 164 to at least the melting point of the material from which the plug is formed. In some instances, the plug 164 may be formed from wax or a wax-like material such as paraffin wax (melting temperature of 55°C to 75°C), bees wax (melting temperature of 62°C), stearin wax (melting temperature of 54°C to 74.5°C) and polyethylene glycol (melting temperature of 51°C to 53°C). In some embodiments, the plug materials may have respective melting points that range from about 50°C to about 80°C, which are temperatures associated with post assembly processes such as gross leak testing and ethylene oxide ("EtO") sterilization. As used herein in the context of temperature, the word about means +/- 5%. For example, the plug material may have a melting point of about 50°C in some implementations. The melting temperature should also be less than the demagnetization temperature of the magnets 112, which may be about 80°C to about 100°C. It should be noted that, due to the distance between the plug 164 and the weld 122, the plug will typically not shrink or melt during the welding process and will remain intact until, for example, a post assembly process.

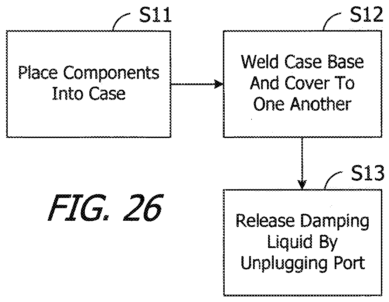

Turning to FIGS. 22 and 23, which respectively show the exemplary magnet assembly 100a and damping liquid dispenser 154 after the port 162 has been unplugged by, for example, heating the plug 164, it should be noted that the damping liquid 116 is gravity fed through the open port. Rotation of the damping liquid dispenser 154 from the orientation illustrated in FIGS. 22 and 23 to, for example, the orientation illustrated in FIGS. 24 and 25 will result in the damping liquid 116 flowing through the port 162, through the recess 166 and into the interior of the case 102. Such rotation may be accomplished by simply reorienting the entire magnet assembly 100a by causing the magnets 112a to rotate about their axes A2 with a magnetic field. Such rotation may occur as part of the manufacturing process and/or after the magnet assembly 100a has been implanted with a cochlear implant. In summary, and referring to FIG. 26, in one exemplary method, the internal components of a magnet assembly, including the magnets and the damping liquid dispenser, may be placed into a case (Step S11), the case base and cover may then be welded to one another (Step S12), and damping liquid may be released from the damping liquid dispenser into the case by unplugging the damping liquid dispenser port (Step S13). Subsequent movement of the internal components of the magnet assembly 100a, i.e., rotation of the frame 108 relative to the case 102 and/or rotation of the magnets 112a relative the case 102, the frame 108 and the tubes 146, will cause the damping liquid to encapsulate the internal components and act as a physical dampening medium between the components.

There are a number of advantages associated with the magnet assembly 100a and associated methods. By way of example, but not limitation, the damping fluid remains within the damping fluid dispenser 154 while case base 104 and cover 106 are being welded to one another, thereby obviating the issues associated with boiling damping fluid during the formation of the weld 122. The use of the damping fluid dispenser 154 also facilitates the use of a conventional case.

It should be noted the examples described above with reference to FIGS. 13-26 may be modified in a variety of ways. By way of example, but not limitation, the number of magnets 112' and 112" may be decreased to three or two or one, or may be increased to five or more. A one-piece frame, such that discussed above, may employed in place of the two-piece frame 108a.

One example of a cochlear implant (or "implantable cochlear stimulator") including the present magnet assembly 100 (or 100a) is the cochlear implant 200 illustrated in FIG. 27. The cochlear implant 200 includes a flexible housing 202 formed from a silicone elastomer or other suitable material, a processor assembly 204, a cochlear lead 206, and an antenna 208 that may be used to receive data and power by way of an external antenna that is associated with, for example, a sound processor unit. The cochlear lead 206 may include a flexible body 210, an electrode array 212 at one end of the flexible body, and a plurality of wires (not shown) that extend through the flexible body from the electrodes 212a (e.g., platinum electrodes) in the array 212 to the other end of the flexible body. The magnet assembly 100 is located within a region encircled by the antenna 208 (e.g., within an internal pocket 202a defined by the housing 202) and insures that an external antenna (discussed below) will be properly positioned relative to the antenna 208. The exemplary processor assembly 204, which is connected to the electrode array 212 and antenna 208, includes a printed circuit board 214 with a stimulation processor 214a that is located within a hermetically sealed case 216. The stimulation processor 214a converts the stimulation data into stimulation signals that stimulate the electrodes 212a of the electrode array 212.

Turning to FIG. 28, the exemplary cochlear implant system 60 includes the cochlear implant 200, a sound processor, such as the illustrated body worn sound processor 300 or a behind-the-ear sound processor, and a headpiece 400.

The exemplary body worn sound processor 300 in the exemplary ICS system 60 includes a housing 302 in which and/or on which various components are supported. Such components may include, but are not limited to, sound processor circuitry 304, a headpiece port 306, an auxiliary device port 308 for an auxiliary device such as a mobile phone or a music player, a control panel 310, one or more microphones 312, and a power supply receptacle 314 for a removable battery or other removable power supply 316 (e.g., rechargeable and disposable batteries or other electrochemical cells). The sound processor circuitry 304 converts electrical signals from the microphone 312 into stimulation data. The exemplary headpiece 400 includes a housing 402 and various components, e.g., a RF connector 404, a microphone 406, an antenna (or other transmitter) 408 and a diametrically magnetized disk-shaped positioning magnet 410, that are carried by the housing. The headpiece 400 may be connected to the sound processor headpiece port 306 by a cable 412. The positioning magnet 410 is attracted to the magnet assembly 100 of the cochlear stimulator 200, thereby aligning the antenna 408 with the antenna 208. The stimulation data and, in many instances power, is supplied to the headpiece 400. The headpiece 400 transcutaneously transmits the stimulation data, and in many instances power, to the cochlear implant 200 by way of a wireless link between the antennae. The stimulation processor 214a converts the stimulation data into stimulation signals that stimulate the electrodes 212a of the electrode array 212.

In at least some implementations, the cable 412 will be configured for forward telemetry and power signals at 49 MHz and back telemetry signals at 10.7 MHz. It should be noted that, in other implementations, communication between a sound processor and a headpiece and/or auxiliary device may be accomplished through wireless communication techniques. Additionally, given the presence of the microphone(s) 312 on the sound processor 300, the microphone 406 may also be omitted in some instances. The functionality of the sound processor 300 and headpiece 400 may also be combined into a single head wearable sound processor. Examples of head wearable sound processors are illustrated and described in U.S. Patent Nos. 8,811 643 and 8,983,102.

Although the inventions disclosed herein have been described in terms of the preferred embodiments above, numerous modifications and/or additions to the above-described preferred embodiments would be readily apparent to one skilled in the art. The inventions include any combination of the elements from the various species and embodiments disclosed in the specification that are not already described. It is intended that the scope of the present inventions extend to all such modifications and/or additions and that the scope of the present inventions is limited solely by the claims set forth below.

## Claims

1. A method of assembling a magnet assembly, comprising:
positioning a plurality of elongate diametrically magnetized magnets (112) within a first case member (104) including a first end wall (124) and a first side wall portion;
securing a second case member (106), including a second end wall (126) and a second side wall portion, to the first case member (104) to form a case (102) defining a central axis (A1) and in which the elongate diametrically magnetized magnets (112) are located, by welding the first side wall portion to the second side wall portion, wherein one of the first and second end walls (124, 126) includes a port (118);
introducing damping liquid (116) into the case (102) by way of the port (118); and
sealing the port (118) after the damping liquid (116) has been introduced into the case (102) by way of the port (118).

2. A method as claimed in claim 1, wherein
sealing the port (118) comprises welding a plug (120) to the end wall that includes the port (118).

3. A method as claimed in claim 1 or claim 2, wherein
the first and second side wall portions together define an annular side wall (128) that is located between the first and second end walls (124, 126); and
the central axis (A1) passes through the port (118).

4. A method as claimed in claim 2 or claim 3, wherein
the plug (120) and the case (102) are formed from the same material.

5. A method as claimed in claim 4, wherein
the plug (120) and the case (102) are formed from titanium.

6. A method as claimed in any one of claims 1 to 5, wherein
the damping liquid (116) comprises a biocompatible oil.

7. A method as claimed in any one of claims 1 to 6, wherein
the damping liquid (116) has a viscosity that is greater than or equal to about 86 centipoise (cps) and is less than or equal to about 150,000 cps.

8. A method as claimed in any one of claims 1 to 7, wherein
the elongate diametrically magnetized magnets (112) are located within a holder (114) that includes a plurality of tubes (146).

9. A method as claimed in any one of claims 1 to 8, further comprising the step of:
positioning a frame (108) within the first case member (104) prior to securing the second case member (106) to the first case member (104);
wherein the frame (108) is rotatable relative to the case (102) about the central axis (A1) of the case (102); and
wherein each elongate diametrically magnetized magnet (112) is located in the frame (108), defines a longitudinal axis (A2) and a N-S direction, and is rotatable about its respective longitudinal axis (A2) relative to the frame (108) and the case (102).

10. A cochlear implant, comprising:
a cochlear lead (206) including a plurality of electrodes (212a);
an antenna (208);
a stimulation processor (214a) operably connected to the antenna (208) and to the cochlear lead (206); and
a magnet assembly (100) produced by the method recited in any one of claims 1-9.

11. A cochlear implant as claimed in claim 10, wherein
the antenna (208), the stimulation processor (214a) and the magnet assembly (100) are located within a flexible housing (202).

12. A system, comprising:
a cochlear implant as claimed in claim 10 or claim 11; and
an external device (400) including a diametrically magnetized disk-shaped positioning magnet (410).

## Patentansprüche

1. Verfahren zum Montieren einer Magnetbaugruppe, wobei:
eine Mehrzahl von langgestreckten diametral magnetisierten Magneten (112) innerhalb eines ersten Gehäusebauteils (104) mit einer ersten Endwand (124) und einem ersten Seitenwandabschnitt positioniert wird;
ein zweites Gehäusebauteil (106) mit einer zweiten Endwand (126) und einem zweiten Seitenwandabschnitt an dem ersten Gehäusebauteil (104) befestigt wird, um ein Gehäuse (102) zu bilden, welches eine zentrale Achse (A1) aufweist und in welchem die langgestreckten diametral magnetisierten Magneten (112) angeordnet sind, indem der erste Seitenwandabschnitt an den zweiten Seitenwandabschnitt angeschweißt wird, wobei die erste oder die zweite Endwand (124, 126) einen Anschluss (118) aufweisen;
Dämpfungsflüssigkeit (116) mittels des Anschlusses (118) in das Gehäuse (102) eingebracht wird; und
der Anschluss (118) abgedichtet wird, nachdem die Dämpfungsflüssigkeit (116) mittels des Anschlusses (118) in das Gehäuse (102) eingebracht wurde.

2. Verfahren gemäß Anspruch 1, wobei beim Abdichten des Anschlusses (118) ein Stopfen (120) an die Endwand geschweißt wird, welche den Anschluss (118) beinhaltet.

3. Verfahren gemäß Anspruch 1 oder 2, wobei der erste Seitenwandabschnitt und der zweite Seitenwandabschnitt zusammen eine ringförmige Seitenwand (12) bilden, welche zwischen der ersten und der zweiten Endwand (124, 126) angeordnet ist, und wobei die zentrale Achse (A1) durch den Anschluss (118) verläuft.

4. Verfahren gemäß Anspruch 2 oder 3, wobei der Stopfen (120) und das Gehäuse (102) aus demselben Material gebildet sind.

5. Verfahren gemäß Anspruch 4, wobei der Stopfen (120) und das Gehäuse (102) aus Titan gebildet sind.

6. Verfahren gemäß einem der Ansprüche 1-5, wobei die Dämpfungsflüssigkeit (116) ein biokompatibles Öl aufweist.

7. Verfahren gemäß einem der Ansprüche 1-6, wobei die Dämpfungsflüssigkeit (116) eine Viskosität aufweist, die größer oder gleich etwa 86 Centipoise (cps) und kleiner oder gleich etwa 150.000 cps ist.

8. Verfahren gemäß einem der Ansprüche 1-7, wobei die langgestreckten diametral magnetisierten Magneten (112) in einem Halter (114) angeordnet sind, welcher eine Mehrzahl von Röhren (146) aufweist.

9. Verfahren gemäß einem der Ansprüche 1-8, wobei ferner:
ein Rahmen (108) innerhalb des ersten Gehäusebauteils (104) positioniert wird, bevor das zweite Gehäusebauteil (106) an dem ersten Gehäusebauteil (104) befestigt wird;
wobei der Rahmen (106) relativ zu dem Gehäuse (102) um die zentrale Achse (A1) des Gehäuses (102) drehbar ist; und
wobei jeder langgestreckte diametral magnetisierte Magnet (112) in dem Rahmen (108) angeordnet ist, eine longitudinale Achse (A2) und eine N-S-Richtung aufweist und drehbar um seine jeweilige longitudinale Achse (A2) relativ zu dem Rahmen (108) und dem Gehäuse (102) ist.

10. Cochlea-Implantat mit:
eine Cochlea-Zuleitung (206) mit einer Mehrzahl von Elektroden (212a);
einer Antenne (208);
einem Stimulationsprozessor (214a), der in Wirkverbindung mit der Antenne (208) und der Cochlea-Zuleitung (206) steht; und
einer Magnetbaugruppe (100), die mittels des in einem der Ansprüche 1-9 genannten Verfahrens hergestellt ist.

11. Cochlea-Implantat gemäß Anspruch 10, wobei die Antenne (208), der Stimulationsprozessor (214a) und die Magnetbaugruppe (100) innerhalb eines flexiblen Gehäuses (202) angeordnet sind.

12. System mit:
einem Cochlea-Implantat gemäß einem der Ansprüche 10 oder 11; und
einer externen Vorrichtung (400) mit einem diametral magnetisierten scheibenförmigen Positionierungsmagneten (410).

## Revendications

1. Procédé d'assemblage d'un ensemble d'aimants, comprenant :
le positionnement d'une pluralité d'aimants allongés magnétisés diamétralement (112) à l'intérieur d'un premier élément de boîtier (104) comportant une première paroi d'extrémité (124) et une première partie de paroi latérale ;
la fixation d'un second élément de boîtier (106), comportant une seconde paroi d'extrémité (126) et une seconde partie de paroi latérale, au premier élément de boîtier (104) pour former un boîtier (102) définissant un axe central (A1) et dans lequel sont situés les aimants allongés diamétralement aimantés (112), par soudage de la première partie de paroi latérale à la seconde partie de paroi latérale, l'une des première et seconde parois d'extrémité (124, 126) comportant un orifice (118) ;
l'introduction d'un liquide d'amortissement (116) dans le boîtier (102) par l'intermédiaire de l'orifice (118) ; et
le scellage de l'orifice (118) après que le liquide d'amortissement (116) a été introduit dans le boîtier (102) par l'intermédiaire de l'orifice (118).

2. Procédé selon la revendication 1, dans lequel le scellage de l'orifice (118) comprend le soudage d'un bouchon (120) à la paroi d'extrémité qui comporte l'orifice (118).

3. Procédé selon la revendication 1 ou la revendication 2, dans lequel
les première et seconde parties de paroi latérale définissent ensemble une paroi latérale annulaire (128) qui est située entre les première et seconde parois d'extrémité (124, 126) ; et
l'axe central (A1) passe par l'orifice (118).

4. Procédé selon la revendication 2 ou la revendication 3, dans lequel
le bouchon (120) et le boîtier (102) sont formés du même matériau.

5. Procédé selon la revendication 4, dans lequel le bouchon (120) et le boîtier (102) sont en titane.

6. Procédé selon l'une quelconque des revendications 1 à 5, dans lequel
le liquide d'amortissement (116) comprend une huile biocompatible.

7. Procédé selon l'une quelconque des revendications 1 à 6, dans lequel
le liquide d'amortissement (116) présente une viscosité supérieure ou égale à environ 86 centipoises (cps) et inférieure ou égale à environ 150 000 cps.

8. Procédé selon l'une quelconque des revendications 1 à 7, dans lequel
les aimants allongés magnétisés diamétralement (112) sont situés à l'intérieur d'un support (114) qui comporte une pluralité de tubes (146).

9. Procédé selon l'une quelconque des revendications 1 à 8, comprenant en outre l'étape suivante :
le positionnement d'un cadre (108) à l'intérieur du premier élément de boîtier (104) avant de fixer le second élément de boîtier (106) au premier élément de boîtier (104) ;
dans lequel le cadre (108) est mobile en rotation par rapport au boîtier (102) autour de l'axe central (A1) du boîtier (102) ; et
dans lequel chaque aimant allongé magnétisé diamétralement (112) est situé dans le cadre (108), définit un axe longitudinal (A2) et une direction N-S, et peut tourner autour de son axe longitudinal (A2) respectif par rapport au cadre (108) et au boîtier (102).

10. Implant cochléaire, comprenant :
un fil cochléaire (206) comportant une pluralité d'électrodes (212a) ;
une antenne (208) ;
un processeur de stimulation (214a) connecté fonctionnellement à l'antenne (208) et au fil cochléaire (206) ; et
un ensemble d'aimants (100) produit par le procédé selon l'une quelconque des revendications 1 à 9.

11. Implant cochléaire selon la revendication 10, dans lequel
l'antenne (208), le processeur de stimulation (214a) et l'ensemble d'aimants (100) sont situés à l'intérieur d'un boîtier flexible (202).

12. Système comprenant :
un implant cochléaire selon la revendication 10 ou la revendication 11 ;
un dispositif externe (400) comportant un aimant de positionnement en forme de disque diamétralement magnétisé (410).
